Europäisches Patentamt

European Patent Office

Office européen des brevets

(11) Publication number: **0 202 472**
**B1**

(12) EUROPEAN PATENT SPECIFICATION

(45) Date of publication of patent specification:
09.08.89

(51) Int. Cl.⁴: **D 21 H 5/26, A 61 L 15/00**

(21) Application number: 86105203.3

(22) Date of filing: 15.04.86

(54) Non-laminated dry formed absorbent product.

(30) Priority: 22.04.85 US 725411

(43) Date of publication of application:
26.11.86 Bulletin 86/48

(45) Publication of the grant of the patent:
09.08.89 Bulletin 89/32

(84) Designated contracting states:
BE CH DE FR GB LI NL SE

(56) References cited:
US—A— 1 599 371
US—A— 4 160 059

(73) Proprietor: GRAIN PROCESSING CORPORATION
1600 Oregon Street
Muscatine, Iowa 52761 (US)

(72) Inventor: Djock, Jeffrey C.
4502 Cedercrest
Portage Michigan 49081 (US)
Inventor: Harvey, Richard D.
Rt. No. 6, Box 53
Muscatine Iowa 52761 (US)
Inventor: Klem, Robert E.
2006 West Bay Drive
Muscatine Iowa 52761 (US)

(74) Representative: Hoeger, Stellrecht & Partner
Uhlandstrasse 14 c
D-7000 Stuttgart 1 (DE)

## Description

The present invention relates to absorbent products and the production thereof.

Absorbent disposable products find numerous uses such as disposable baby and adult diapers, underpads, catamenial devices, wound and surgical dressings and wipes. In addition to sanitary and medical applications, absorbent products find use as filter devices or absorbent pads in applications where there is need to absorb fluid contaminants such as in motor fuel, transformer fluids and hydraulic fluids and as disposable industrial toweling for spills and so forth. The main function of such products is to absorb and retrain fluids. These products are usually constructed as laminates wherein an absorbent material is disposed between one or more lamina of liquid permeable sheets and in some cases one or more liquid impermeable sheets.

In the last few years certain hydrocolloidal polymers have been developed which have the ability to absorb large quantities of liquids, including, for example, body fluids. The use of these absorbents in disposable softgoods products such as diapers, wound dressings, catamenial tampons and the like offers advantages. When used in disposable softgoods products these materials result in better absorbency, thereby permitting reduction in the bulk of the disposable product by allowing the manufacturer to decrease the amount of cellulose wadding or fluff pulp in the disposable product.

Many procedures have been suggested in an effort to prepare absorbent products and a number of web formed absorbent laminates have been suggested such as disclosed in United States patent No. 4 117 414 (film-tissue laminates) and in United States patent No. 4 260 443 (paper-tissue laminates).

Such laminates are usually formed by moistening the absorbent film or post wetting the laminate structure. Film laminates are formed at relatively low line speeds, such as 80-100 feet per minute (24.4-30.5 meters per minute), due to high amounts of water inherent in the casting operation and require additional treatments such as, for example, crushing as disclosed in United States patent No. 4 293 609, to make them satisfactory for many applications. Post wetting of an embossed laminate structure using a spot application of water results in a product which tends to delaminate on further converting operations (i. e., rewinding and slitting). The resultant product also retains the undesirable properties of rattle and coarse texture.

The prior art processes for producing absorbent products admit of certain disadvantages. For example, certain prior art manufacturing procedures require special carrier sheets to which the absorbent is applied and/or the use of particular solvent systems to avoid water swell of the absorbent during manufacture. Also, prior art processes involve intricate systems to apply and retain the absorbent in the laminate product with the result being that such manufacturing procedures are quite complicated. Often the resulting product is easily delaminated with impaired absorbent properties.

There are additional problems associated with the use of the hydrocolloid polymers in disposable sanitary products. For example, when the hydrocolloidal polymers, especially in powder form, are added to disposable softgoods, the polymers are dusty during application and shift in the finished product. The shifting of the hydrocolloidal particles takes place during the manufacture, handling, storage and use of the article. This can result in the particles bunching together and failing to be evenly distributed throughout the article. United States patent No. 4 260 443 addresses these problems by having the lamina secured together at spaced points with the majority of the absorbent particles located in pockets between such adhering points. Even here, however, the absorbent can accumulate in the pockets and not provide uniform distribution throughout the laminate.

A further disadvantage with laminated or space locking is a loss or weeping of the absorbent material containing the absorbed liquid lending a product which is less effective, uncomfortable and aesthetically unattractive.

It is a major object of the present invention to provide a method by which high quality absorbent products can be prepared by a simple integrated process.

It is a further object of the invention to provide a process for producing an absorbent product in web or sheet form having a liquid absorbing material uniformly distributed throughout the product.

It is a still further object of the invention to produce a process for producing an absorbent product in sheet or web form having desired qualities of strength, liquid absorption, appearance or hand, low dusting and linting characteristics.

A further object of the invention is the provision of a non-laminar absorbent product containing a liquid-absorbing material which is essentially homogenous in structure with no discernable layering of the product.

A further object of the invention is the provision of an absorbent product which upon use effectively retains the absorbed material without excessive weeping or loss.

The present invention provides a non-laminar absorbent product in web or sheet form having a liquid absorbing material, preferably a super liquid absorbing material, distributed substantially homogeneously throughout the product. The absorbent is essentially non-layered and exhibits substantially no dusting or linting characteristics with the liquid absorbance being substantially uniform in all parts of the absorbent web.

The present invention comprises a non-laminar (non-laminated) absorbent product comprising matrix fibers having distributed throughout a liquid-absorbing material and a heat-activated binder material to bind the matrix fibers and liquid-absorbing material into a coherent web.

The absorbent products according to this invention are made by forming in dry condition a sheet or web from matrix fibers which can be cellulosic fibers alone or a mixture of cellulosic and synthetic fibers and a liquid-absorbing material. Thus, according to the present invention, a non-laminated absorbent product is produced by forming in dry form a mat from matrix fibers and distributing among the matrix fibers in the mat in dry form a liquid-absorbing material. A heat activated binder is incorporated in the mat and the mat is heated to a temperature to activate the binder after which the binder thermosets to produce a non-laminar absorbent product in which a liquid-absorbing material is substantly homogeneously integrated therein. In one preferred embodiment of manufacture, the matrix fibers, preferably mixed with a thermoplastic or thermosetting binder, and a liquid-absorbing material are deposited simultaneously on a foraminous web-forming device and thereafter subjected to a heating or calendering operation, or both, to produce a coherent, absorbent product in sheet or web form. Quite unexpectedly, the liquid-absorbent particles, while integrally dispersed and bound throughout the product, exhibit absorption capacities substantially equivalent to the free-swell absorption capacity thereof.

The invention involves a process for producing an absorbent product by forming in substantially dry condition a mat from non-women matrix fibers and distributing in dry form among the non-woven matrix fibers in said mat a liquid-absorbing material. A heat-activated thermoplastic or thermosetting binder material is incorporated in the mat, which in the thermally set state functions to bond said matrix fibers and liquid-absorbing material into a coherent web. The mat is then heated to a temperature sufficient to activate said binder, after which the binder is thermally set to bind the matrix fibers and liquid-absorbing material into a coherent, absorbent product.

The fibers used to form the basic matrix of the absorbent products of the invention include cellulosic fibers such as wood pulp, bagasse, hemp, jute, esparto, rice, wheat, bamboo, corn, sisal, cotton, flax kenaf and the like, and mixtures of different cellulosic fibers. Blends of cellulosic fibers and synthetic staple fibers, such as polyolefins, polyamides, polyacrylamides, polyacrylics, polyesters and the like, can also be used to form the fiber matrix. Generally the matrix-forming fibers are between 0.03 and 3 inches (0.08-7.6 centimeters) in length, and preferably 0.1 to 1.5 inches (0.25 to 3.8 centimeters) in length. Longer fibers can be used dependent upon the ability of the forming equipment to suitably handle long fibers. Usually, the synthetic staple fibers will have greater length than the cellulosic fibers and will be 1/2 inch (1.3 centimers) and more in length. One preferred blend of matrix fibers is a mixture of approximately 95 % by weight of softwood kraft pulp and about 5 % polypropylene fibers. When a combination of cellulosic fibers and staple synthetic fibers is used as the matrix fibers, it is generally preferred that the cellulosic fibers constitute from about 75 to 95 % by weight of the mixture. The synthetic staple fibers, some of which may be of thermoplastic nature, should, in order to maintain their integrity in the finished product, have relatively high softening or melting points above the activating temperature of the binders which are utilized to bind the matrix fibers and liquid-absorbing agent. Generally speaking, the matrix fibers employed will have softening temperatures above about 340 °F (171 °C), however it is only necessary that the temperature be substantially above the activation temperature of the bonding agent.

The binder used in producing the absorbents according to the invention is a thermoplastic or thermosetting material which can be heat activated and thermally set to bind the matrix fibers and particles of liquid-absorbing material into an integral coherent web having sufficient tensile strength to permit its use as an absorbent in such applications as mentioned heretofore. Thus, the binder can be a thermosetting binder of the type which when activated by heating becomes set or hardened. Various types of thermosetting binders are known to the art such as polyvinyl acetate, vinyl acetate, ethylene-vinyl chloride, styrene butadiene, polyvinyl alcohol, polyethers and the like. Also, the binder can be a thermoplastic binder which upon activation by heat becomes soft but which reverts to its normal frozen state upon cooling. Representative of such thermoplastic binder materials are polypropylenes, polyethylenes, polycarbonates, polyvinylchloride, polyesters, polystyrenes, acrylics and the like.

It is generally preferred to employ a thermosetting or thermoplastic binder which is activated, i. e., which becomes set or which becomes soft at a relatively low temperature, but above normal ambient temperature. Thus, the binder is preferably one which is activated at a temperature in the range of 200 to 350 °F (93-177 °C).

Solid binders should be used in a form which facilitates dispersion throughout the fiber matrix. Thus, when a fibrous binder compressed in the form of a roll or sheet is utilized, such binders should be subjected to defribation similar to the matrix fibers. Thermoplastic binders in the form of powders, such as polyvinyl alcohol and polyesters, can also be used. Likewise, a thermosetting binder can be applied in the form of a liquid solution or dispersion. In any case, the binder is generally utilized in amounts ranging from 5 to 50 % by weight of the matrix fiber content and preferably from 10 to 20 % by weight thereof.

Various thermosetting or thermoplastic binders well known in the art which in thermally set state bind the matrix fibers can be used in accordance with this invention. Illustrative thermoplastic binders which are available commercially and which are of fibrous nature include « Pulpex™ E, P and H » (a trademark of a Hercules Incorporated company, Research Center Wilmington, Delaware, 19899), « Kodel 410 » and « Kodel 438 » (TM) available from Eastman Chemical Products, Inc., subsidiary of Eastman Kodak Company, Kingsport, Tennessee 37662, Chisso ES (TM) Fiber available from Nichimen America Inc., 1185

3

Avenue of the Americas, New York, New York 10036, Synthetic Wood Pulp E Grades from Schwarzwalder Textile Works, Heinrich Kutzman GmbH, D-7623 Schenkenzell (Schwarzwald), Swiss Polyamid Grilon (TM) types K115 and K140 from EMS-Grilon, Grilon SA, CH-7013 Domat/Ems, Switzerland, and the like.

Commercially available thermoplastic binders in powder or particulate form include polyvinyl alcohol and polyesters such as « Kativo » (TM) available from H.B. Fuller Co., Minneapolis, Minnesota and the like. Representative thermosetting binder materials which are adapted for application in the form of a liquid dispersion include styrene-butadiene latex emulsions, copolymers of ethylene and acrylic acid, vinyl acetate-ethylene copolymers, acrylonitrile-butadiene copolymers, vinylchloride polymers, vinylidene chloride polymers, « Airflex » (TM) available from Air Products & Chemicals, P.O. Box 97, Calvert City, Kentucky 42029, and the like.

The liquid absorbing materials used in the absorbent products are granular or powdered materials which have the ability to absorb liquids, including body fluids. These absorbents are generally hydrophilic polymeric materials which exhibit some adhesive properties when moistened with water. Hydrophilic polymers which exhibit the ability to absorb large quantities of liquids, i. e. considerably in excess of 15 parts of liquid per part thereof, are often referred to in the art as superabsorbents and generally fall into three classes, namely, starch graft copolymers, crosslinked carboxymethylcellulose derivatives and modified hydrophilic polyacrylates. It is preferred to employ a super absorbent in the absorbent products of this invention. Many such liquid absorbing materials known to the art can be used, including, for example, such materials as disclosed in United States patents Nos. 3 661 815 (Grain Processing Corporation's Polymer 35-A-100, now sold under the tradename « Water Lock »™), 3 935 099, 4 076 663, 4 090 013 and 4 293 609. For example, there can be used carboxylated cellulose, hydrolyzed acrylonitrile-grafted starch, acrylic acid derivative polymers, polyacrylonitrile derivatives, polyacrylamide type compounds and saponified vinyl acetate/methyl acrylate copolymers. Among these polymers, acrylic acid derivative copolymers are especially preferred. These polymers are marketed under trademark « Sanwet » (supplied by Sanyo Kasei Kogyo Kabushiki Kaisha) and « Sumika Gei » (supplied by Sumitomo Kagaku Kabushiki Kaisha). Depending upon the absorbent capacity desired in the product, any liquid absorbing material is generally employed in amounts up to 50 % by weight of the matrix fibers and preferably in an amount of from about 15 to 35 % depending upon the use requirements.

Presently preferred embodiments of the process for making the absorbent products of the invention will low be described in conjonction with the drawings wherein :

Figure 1 is a simplified schematic illustration showing a suitable arrangement of apparatus for producing the absorbent products according to one preferred embodiment.

Figure 2 is a graphical plot comparing the absorbency efficiency of an absorbent product prepared in accordance with the invention with the liquid absorbing agent Water Lock A-220 in free state, i. e. not bound with matrix fibers (free swell).

Figure 3 is a graphical plot comparing the absorbency efficiency of an absorbent product prepared in accordance with the invention with the liquid absorbing agent Water Lock A-220 in free state, i. e. not bound with matrix fibers (free swell).

Figure 4 is a graphical plot of saline absorption of a product produced in accordance with the invention compared with the saline absorption of the liquid absorbing agent Water Lock A-220 in free state (free swell).

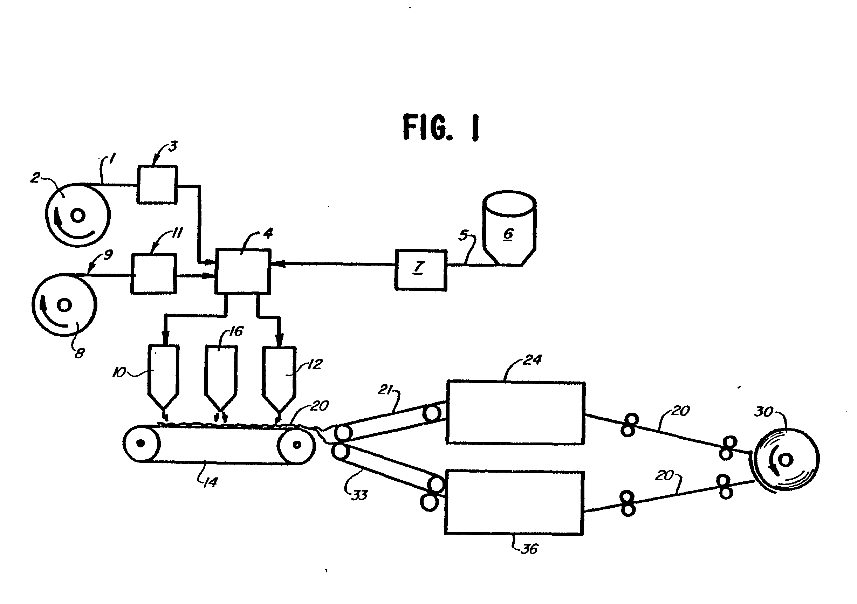

Conventional equipment known in the art for making air-laid paper or cellulosic toweling can be utilized and variously arranged. Thus, with reference to Figure 1 of the drawing, a roll of a cellulosic fibrous material 1, such as softwood kraft pulp, is unwound from stand 2 and passed to a defibrating device 3 wherein the cellulosic paper is opened to produce a dry fluffy mass. Defibrating equipment is commercially available for opening the cellulosic fibers. The dry fluffed cellulosic fibers are then passed to blending device 4. Optionally, a mixture of different fibrous materials can be used as the base or matrix-forming fibers and such other fibrous materials can be handled similarly. Thus, in such an optional multiple fiber embodiment, a staple fiber 5 such as a relatively high melting point polyolefin, for example polypropylene, is supplied by fiber feed device 6 and, if necessary, passed through a fiber opening device 7 and to blending device 4. A fibrous thermoplastic binder generally supplied in bale or bulk tow is used. Thus, illustratively, a relatively low melting point polyethylene 9 is supplied similarly from a fiber feeder 8 and subjected to defibration or opening at 11 from which the fluffed polyethylene is sent to blending device 4. In the blending device 4 the primary cellulosic fibers 1 and the staple fibers 5 and the binder fibers 9 are intimately admixed. Preblenbed mixtures of multiple matrix fibers can be used when available. The admixed or blended fibers pass from blender 4 to two forming heads 10 and 12 which can be conventional forming heads or fiber laying devices of known type which are used in the art for air laying fibers to form paper sheets or cellulosic tissues. The matrix-forming fluffed fibers are deposited from forming heads 10 and 12 onto a forming unit 14. Forming unit 14 is of the conventional type for air-laying fibers in mat form. Such equipment is available commercially from suppliers such as Dan Webforming, Bryggervej 21, DK-8240 Risskov, Aurhus, Denmark, Rando Machine Corp., The Commons, Macedon, New York 14502. Such forming devices generally utilize either suction or air so as to distribute and arrange the matrix fibers in the form of a non-cohered, non-integrated mat.

Disposed between forming heads 10 and 12 is a powder scattering device 16, which serves to distribute a metered amount of a dry, granular or powdered liquid absorbent material onto the mat of matrix fibers. The powder scattering device 16 can be a vibrating feeder, dry sprayer, drum or roll feeder or electrostatic deposition device. The composite mat 20 comprised of the matrix fibers, the binder fibers and liquid-absorbing material leaving the forming unit 14 is transferred by means of either conveyor 21 or 33 to a binder activating operation. Activation of the binder is accomplished by heating to soften and tackify a thermoplastic binder and to cause it to adhere to the matrix fibers and bond them and the liquid-absorbing material into a coherent web. If a thermosetting binder is employed, the heating causes the binder to set and likewise form a coherent web. This heating of the mixture can be accomplished by passing the composite mat 20 via conveyor 21 to air dryer 24 of known type. In air dryer 24, the web is heated to a temperature sufficiently high to melt a thermoplastic binder and set a thermosetting binder but insufficient to affect adversely the matrix fibers or the liquid-absorbing agent. Thus, the temperature to which the composite mat is heated in dryer 24 will depend upon the type of matrix fibers utilized and the particular liquid-absorbing agent employed as well as the thermal characteristics of the particular binder employed. The heating temperature to be employed can be readily determined. Generally speaking, temperatures on the order of 220° F. to 360° F. (104° C. to 182 ° C.) can be employed in dryer 24. Thermal bonding units or air dryers include equipment manufactured by Honeycomb Systems Inc., Box 502, Biddeford Maine 04005, Web Systems — Division of Thermo-Electron, P.O. Box 568, 211 Jackson Street, Neenah, Wisconsin 54956, Fleissner, Inc., Route 5, Box 1005, Charlotte, N.C. 28208, Ramisch Kleinewefers GmbH, Neuer Weg 24, 4150 Krefeld, West Germany, Mohr Machinen & Apparatebau GmbH, Gesellschaft mbH & Co., Landauer St. 21-23, Gerabronn 7182, West Germany, Eduard Kusters Maschinenfabrik, Gladbacher Str. 457, D-4150 Krefeld, Germany and Brucckner Truckentechnik GmbH & Co., Benzstrasse 8-10, D-7250 Leonberg, Germany. After leaving the air dryer 24, the mat cools and the thermoplastic binder reverts to the frozen state so as to bind the matrix-forming fibers and liquid-absorbing material into a coherent, substantially homogeneous web. The adsorbent web can then be collected on storage reel 30 and is ready for cutting, packaging, etc.

In lieu of heating the mat 20 in air dryer 24, activation of the binder can be accomplished by passing the non-cohered mat 20 leaving the web former 14 via conveyer 33 to a calendering operation at 36. Conventional calender equipment can be used to apply sufficient pressure and heat so as to activate the binder and to result in bonding. Exemplary of such suitable calendering equipment are those produced by B.F. Perkins, a division of Rochlen Industries, 939 Chicopee Street, Chicopee, Mass. 01021, Fleissner, Inc., Route 5, Box 1005, Charlotte, N.C. 28208, Ramisch Kleinewefers BmbH, Neuer Weg 24, 4150 Krefeld, West Germany, Rodney Hunt, Hunt, Rodpney Co., 76 Water Street, Orange, Mass. 01364 and Duotex, Galleria Buenos Aires 16, 20124 Milano, Italy. The calendered web 20 leaving the calendering operation 36 is collected on storage reel 30.

If desired, the non-cohered mat leaving web former 14 can be subjected to finishing by a combination of both heating and calendering. In such case, the air dryer 24 and calender 36 can be arranged in series with the mat leaving air dryer 24 being sent to calender 36.

In the process described in Figure 1, the matrix forming fibers are deposited on the web-forming device 14 at two locations (10 and 12) with the liquid-absorbing material being deposited at an intermediate location (16). This manner of forming the absorbent web is most preferred for many end use applications since the liquid-absorbing material is distributed between the matrix fibers deposited from forming devices 10 and 12. This assures that the liquid absorbent is not exposed on one surface of the finished absorbent web.

It is known to those skilled in the art that fibers including cellulosic, staple and binder fibers are available in many different forms such as in the form of rolls, laps, bales, fluffs and tows. Accordingly, the apparatus employed to form the absorbent products can be adapted to accomodate the materials used as is well known to the art.

The following examples further illustrate the invention and the advantages thereof.

## Example 1

Handsheets were produced by laying down on a wire a mat of defribated cellulosic fibers (approximately 48.9 g/m$^2$ (30 pounds per 3 000 ft.$^2$ ream)). A superabsorbent in powdered form was sprinkled on the mat and then additional matrix fibers were laid thereon. The superabsorbent was employed in an amount to provide 4 grams/ft.$^2$ (43 grams/m$^2$). The matrix fibers were sulfite softwood fibers (75 %) and 25 % hardwood. A binder fiber, Pulpex$^{TM}$ E338 (polyethylene), was admixed with the matrix fiber in an amount of 15 % by weight thereof.

The dry formed mat was then placed between two sheets of aluminum foil and heat pressed using a Noble and Wood hot plate to form the test sample. A control mat was prepared in the same manner except no superabsorbent was added. Two test swatches of 5 × 10 cm (2 × 4 inches) were cut from the control. One swatch (Control I) was tested for absorbency without further alteration. A second swatch (Control II) was sprinkled with powdered superabsorbent to give a superabsorbent loading of 43 g/m$^2$ (4 grams/ft.$^2$). These two control swatches (one with no superabsorbent (Control I) and one with superabsorbent sprinkled on top (Control II) and a swatch from the test sample handsheet prepared with bound superabsorbent (Test Sample) were tested for ability to absorb 1 % solution of sodium chloride. Results* obtained on testing are as follows :

|  | Sample Weight | Saline Absorption, 2 min. |
|---|---|---|
| Test Sample | 0.76 g | 11.1 mls |
| Control I | 0.52 g | 9.5 mls |
| Control II | 0.79 g | 14.4 mls |

Control I — Dry Formed Sheet — No Superabsorbent

Control II — Dry Formed Sheet — 4 g. Superabsorbent/ft.$^2$ (43 g/m$^2$) (Applied by sprinkling, not bound, to allow free swell.)

* (1) A dry formed sheet with no superabsorbent is a control for absorbency of sheet alone (Control I)

(2) A dry formed sheet with free (unbound) superabsorbent added reflects the total uptake (dry formed sheet plus free superabsorbent( (Control II)

(3) The difference between Control I and Control II reflects the absorbency of the superabsorbent at 100 % efficiency

(4) The test sample (dry formed containing bound superabsorbent) reflects the performance of the bound superabsorbent.

## Example 2

Absorbent webs were dry formed on a Rando-Webber® * utilizing various compositions of matrix fibers and binders. The binders were used in an amount corresponding to 15 % by weight of the matrix fibers. The superabsorbent (WATER LOCK™ A-205 sold by Grain Processing Corporation) was distributed at a level of 4-5 grams/ft.$^2$ (43-54 grams/m$^2$) and matrix fibers deposited on the mat after applying the superabsorbent. The mats were then bonded as shown in Table I below. The bonded absorbent webs were tested for absorbing performance using a solution of synthetic urine of water. The results are shown in Table I.

The products prepared had superabsorbent distributed substantially homogeneously throughout the web and did not exhibit dusting. The results illustrate that an absorbent sheet can be prepared by dry form processing which exhibits good hand and strength, is free from superabsorbent dusting and substantially retains the absorbent qualities of the superabsorbent.

* A registered trademark of Rando Machine Corp.

(See Table page 7)

| Sample | Base Fibers | | Binder | Bonding Conditions | Sample weight | Absorbency | | Tensile KN/M (#/in.) |
|---|---|---|---|---|---|---|---|---|
| 1 | Swd BK[a] Staple[b] | 90 % 10 % | Latex Spray | | 2.65g | 26.8 mls | S.U.[d] | 0.7 ( 4.0) |
| 2 | Swd BK Staple | 80 % 5 % | Kodel 410 (15 %) | Thermal 147 °C (297 °F) | 1.44 | 23.9 | S.U. | 0.82 ( 4.7) |
| 3 | Swd BK Staple | 80 % 5 % | Kodel 410 (15 %) | Thermal 169 °C (337 °F) | 1.46 | 18.9 | S.U. | 1.82 (10.4) |
| 4 | Swd BK Staple | 80 % 5 % | Pulpex E-338 (15 %) | Thermal 169 °C (337 °F) | 1.47 | 19.3 | S.U. | 1.5 (8.6) |
| 5 | Swd BK Staple | 80 % 5 % | Pulpex E-338 (15 %) | Calender 132 °C (270 °F) 300 PLI[f] 30 fpm | 1.43 | 14.2 | S.U. | N.T.[c] |
| 6 | Swd BK Staple | 80 % 5 % | Kodel 410 (15 %) | Calender 132 °C (270 °F) 300 PLI 30 fpm | 1.09 | 15.1 | S.U. | N.T. |
| 7 | Swd BK Staple | 80 % 5 % | Pulpex E-338 (15 %) | Calender 118 °C (245 °F) 300 PLI 30 fpm | 0.95 | 16.2 | S.U. | N.T. |
| 8 | Swd BK Staple | 80 % | Kodel 416 (15 %) | Calender 118 °C (245 °F) 300 PLI 30 fpm | 1.27 | 26.8 | W.[e] | N.T. |
| 9 | Swd BK Staple | 80 % 5 % | Kodel 410 (15 %) | Through-Air 147 °C (297 °F) 35 fpm | 1.52 | 36.2 | W. | N.T. |
| 10 | Swd BK | 80 % | Kodel 410 (15 %) | Through-Air 169 °C (337 °F) | 1.58 | 30.3 | W. | N.T. |

[a]Swd BK — Softwood Bleached Kraft
[b]Staple — Polypropylene
[c]N.T. — Not Tested
fpm — foot per minute
 Metric — meters per minute
 30 = 9.1 m/min.
[d]S.U. — Synthetic Urine
[e]W. — Distilled Water
[f]PLI — Pressure (Pounds Per Linear Inch)
 Metric — N/m
 I PLI = 175 N/m

## Example 3

Sheets were prepared using the Dan-Webforming pilot former from bleached softwood kraft and Chisso (a bicomponent binder fiber of polypropylene and polyethylene) at a ratio of 75/25, respectively.

Using the fiber composition as described, a mat was laid down. To that mat a superabsorbent powder (WATER LOCK™ A-220) was evenly distributed. A second mat of like composition was laid down and placed over the initial mat superabsorbent structure. The superabsorbent was used in an amount corresponding to about 25 % by weight of the fibers.

The combined sheet structure was bonded using various combinations of heat and embossing.

Examination of the sheets revealed a product which was essentially homogeneous. The superabsorbent was distributed throughout and no discernible layers were present.

Samples of the sheets were tested for absorbent quality by the Demand Absorbency Test using 1 % saline. This test is based on the fact that when exposed to a reservoir of fluid contained within a saturated pad maintained at close to saturation by exposure to free fluid, absorbent materials will draw fluid from that reservoir at a gradually decreasing rate until an equilibrium is established between the saturated pad and the absorbent depending on the composition of the test fluid. The rate and absorptive capacity at equilibrium can be used as an indication of the liquid absorbing effectiveness of particular absorbents. The test is conducted as follows : 300 grams of test fluid are added to a 22.9 cm (9-inch) pie plate. An absorbent pad (Kotex (TM) (regular), femine napkin) is placed in the pie plate and the pad is permitted to become saturated. A Cerex (TM) (Monsanto) nylon nonwoven strip of known weight is placed on the saturated pad. The absorbent composition to be tested is placed on the saturated pad. The absorbent composition to be tested is placed on the Cerex strip and gently brushed to initiate contact. The Cerex strip containing the absorbent composition is removed and weighed at desired time intervals. The test is conducted at a temperature of 70 °F (21 °C and 50 %) relative humidity.

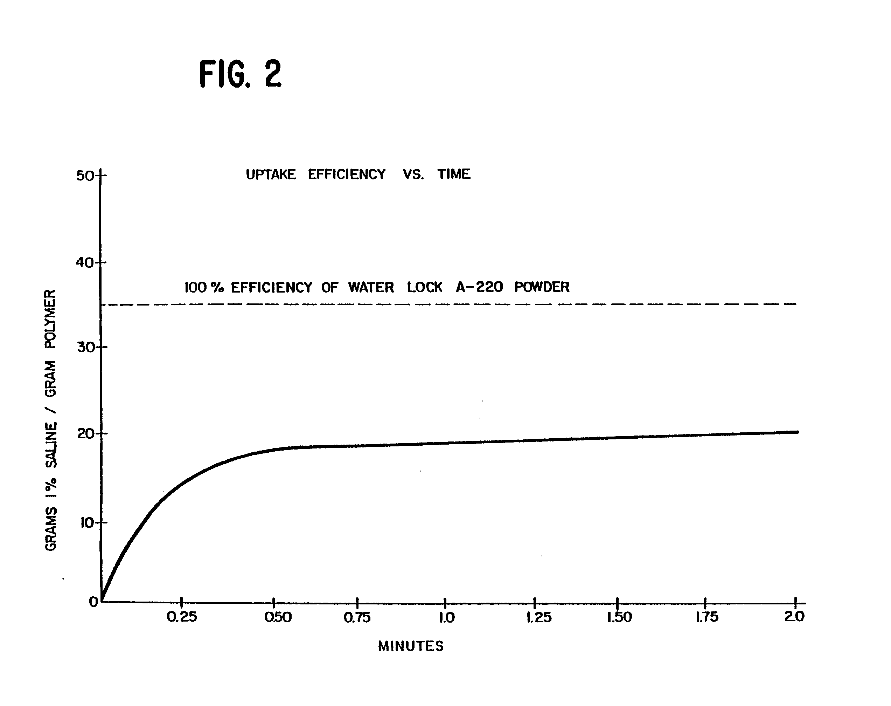

The data (solid line) plotted in Figure 2 compares the absorbent efficiency of the samples to a control containing an equivalent amount of unbound WATER LOCK™ A-220. Upon testing in the saline solution, delamination did not occur.

## Example 4

Sheets were prepared as described in Example 3 containing superabsorbent powder (WATER LOCK™ A-220). The binder fiber (Chisso), however, was reduced to 15 % of the fiber composition and the bonding temperature reduced.

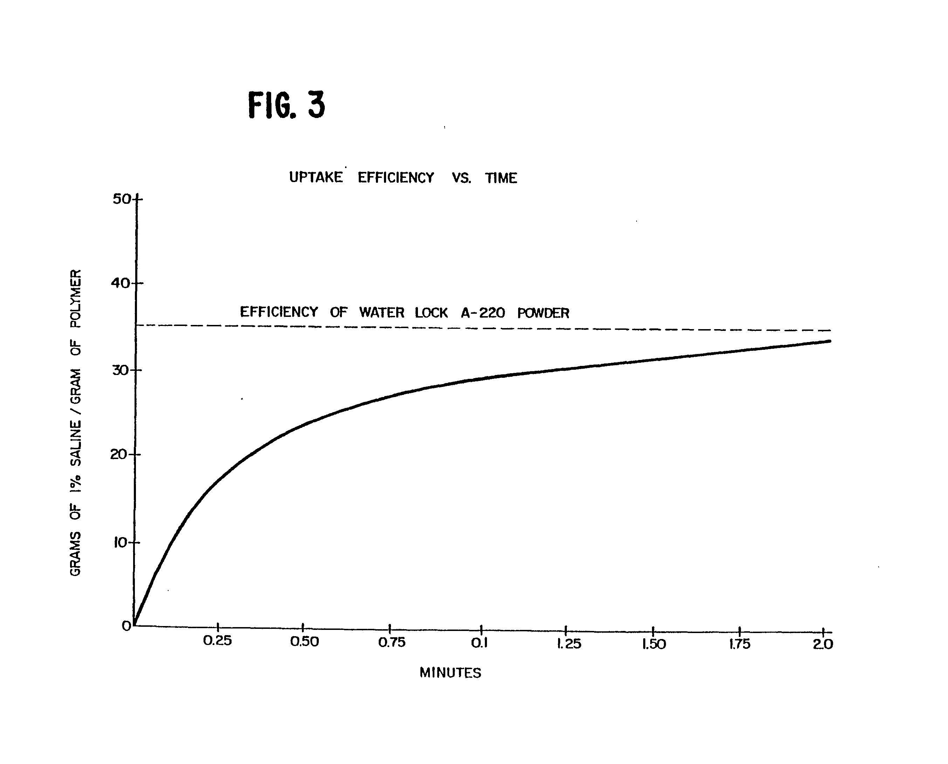

The samples (solid line) were tested for absorbent properties by the Demand Absorbency Test and the results plotted in Figure 3.

The sheets exhibited good strength and were essentially homogeneous with superabsorbent distributed throughout and no discernable layering.

## Example 5

Sheets were prepared using a combination of Dan-Webforming Drum-Former and auxiliary pilot facilities.

A web was laid down of a fiber composition of Southern Pine Bleached Kraft and binder fiber (Pulpex H-231 at a ratio of 85/15, respectively.

Between the two forming heads, superabsorbent powder was applied by a powder feeder (Oxy-Dry) (TM) at a level equivalent to 25.8 g/m² (2.4 grams per square foot) based on the settings of the feeder apparatus.

The combined structure was compacted without heat being applied.

The compatected web was bonded by passage through a pilot dryer at a temperature of 180 °C (365 °F).

The resultant sheet had good appearance, hand, strength and absorbent properties.

Test results on the sheet are as follows :

| | |
|---|---|
| Basis Weight | 135 g/m² (83 #/ream) |
| Tensile | 0.149 KN/m (386 grams/inch) |
| Caliper | 0.318 mm (12.5 × 10⁻³ inches) |

The product was essentially homogeneous with superabsorbent distributed throughout and substantially free from dusting and linting. The product exhibited no discernable layers.

A sample was tested for liquid uptake of saline solution by drawing from a supply vessel with essentially zero differential pressure. The change in weight (liquid uptake) was measured by an Instron (TM) load cell.

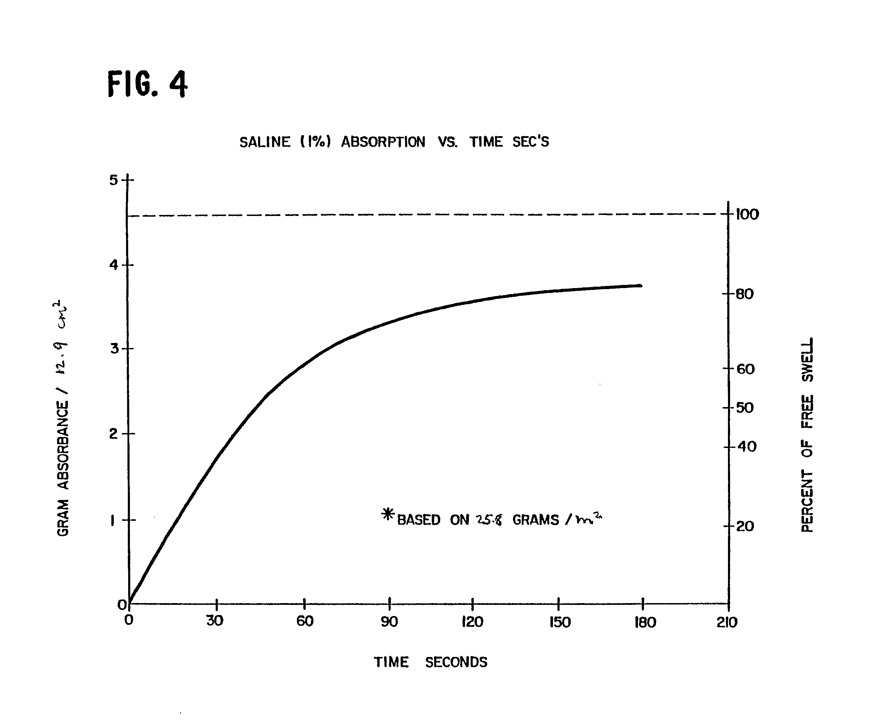

The results are presented graphically in Figure 4 in comparison to the free swell (unbound absorbent) of an equivalent amount of superabsorbent powder (dotted line). As is seen, the product exhibited absorbent efficiency in excess of 80 %. Upon testing in the saline solution, delamination did not occur.

## Example 6

A product was prepared by press bonding at 170 °C for 30 seconds a composition containing 80 % by weight wood pulp and 15 % polypropylene binder and 5 % rayon. No absorbent material was utilized in the product. The product was tested for liquid uptake as a function of time.

The results are shown below :

| | Uptake Grams | | |
| --- | --- | --- | --- |
| Time | 15 seconds | 60 seconds | 120 seconds |
| Apparatus Alone | 1.3 | 2.3 | 3.0 |
| Test Product | 1.15 | 1.7 | 2.35 |

As seen, the polypropylene binder had a negative effect upon absorbency in the absence of an absorbent material.

The advantages of the present invention are significant. Absorbent products can be readily produced in which a liquid-absorbing material is uniformly distributed and bound therein. The liquid-absorbing material does not become displaced or shift during storage or during use. Dusting and linting problems associated with prior art laminated absorbent products are greatly reduced and, since the absorbent is not a laminated structure, delaminating problems are not encountered. By selection of the appropriate compositions (e. g., fiber, binder fibers, staple fibers, and superabsorbents) and handling conditions, one can control the rate of absorbency as well as total absorbent capacity. In addition, this process can be carried out in one continuous operation utilizing relatively basic raw materials to produce a variety of absorbent products.

## Claims

1. A dry-formed absorbent material in the form of a non-laminated coherent web containing a matrix of non-woven cellulosic fibers or a blend thereof with synthetic fibers, a liquid-absorbing material and a thermally set heat-activated binder the liquid-absorbing material being distributed throughout said matrix and bound therein by said heat-activated binder material in thermally set state, characterized in that the non-woven cellulosic fibers constitute a major proportion of the absorbent material, the liquid-absorbing material is capable of absorbing in excess of 15 parts by weight of liquid per part thereof, and the thermally set heat-activated binder constitutes a minor proportion of the absorbent material.

2. An absorbent product according to claim 1 wherein the matrix fibers are cellulosic fibers alone.

3. An absorbent product according to claim 1 wherein the matrix fibers are a mixture of cellulosic fibers and synthetic staple fibers.

4. A absorbent product according to claim 3 wherein the cellulosic fibers constitute at least 75 % by weight of the matrix fibers.

5. An absorbent product according to claim 3 wherein the matrix fibers are comprised of 95 % softwood kraft polymer and 5 % polypropylene.

6. An absorbent product according to claim 1 wherein the binder material is a thermosetting binder material.

7. An absorbent product according to claim 1 wherein the binder material is a thermoplastic binder material.

8. An absorbent product according to claim 1 wherein the liquid-absorbing material is employed in an amount up to 50 % by weight of the matrix fibers.

9. A process for producing an absorbent material according to one of the claims 1-8 which comprises :
   (a) forming in substantially dry condition a mat from the non-woven matrix fibers,
   (b) distributing in dry form amont the non-woven matrix fibers in said mat the liquid-absorbing material,
   (c) incorporating into said mat the thermally setting binder material which in the thermally set state functions to bond said matrix fibers and liquid-absorbing material into a coherent web,
   (d) heating the composition of step (c) to a temperature sufficient to activate said binder, and
   (e) thermally setting said binder material to bind said matrix fibers and said liquid-absorbing material into a coherent, absorbent product, while retaining substantially the absorbing capacity of said liquid-absorbing material.

10. A process in accordance with claim 9 wherein the matrix fibers are composed of a mixture of cellulosic and synthetic fibers.

11. A process in accordance with claim 9 wherein step (d) is conducted together with the application of pressure.

12. A process in accordance with claim 9 wherein the thermoplastic or thermosetting binder is blended with said matrix fibers to forming a mat therefrom.

13. A process in accordance with claim 13 wherein the binder is a fibrous binder material.

14. A process in accordance with claim 12 wherein the binder is a powdered material.

15. A process in accordance with claim 9 wherein a thermoplastic binder is employed.

16. A process in accordance with claim 9 wherein a thermosetting binder is employed.

17. A process in accordance with claim 16 wherein the binder is applied to the mat containing matrix fibers and liquid-absorbing material in a liquid carrier.

18. A process in accordance with claim 9 wherein in step (a) a mat is formed by laying down the matrix fibers on a web-forming device at two or more locations.

19. A process in accordance with claim 18 wherein a liquid-absorbing material is distributed among the matrix fibers in the mat at a location intermediate said locations at which the matrix fibers are laid down.

## Patentansprüche

1. Ein trocken-geformtes, absorbierendes Material in Gestalt einer ungeschichteten, zusammenhängenden Bahn mit einem Gehalt an einer Matrix aus nicht-gewobenen Zellulosefasern oder aus einer Mischung solcher Fasern mit synthetischen Fasern, an einem flüssigkeitsabsorbierenden Material und an einem thermisch ausgehärteten, wärmeaktivierten Bindemittel, wobei das flüssigkeitsabsorbierende Material durch die Matrix hindurch verteilt und darin durch das wärmeaktivierte Bindemittel in thermisch ausgehärtetem Zustand gebunden ist, dadurch gekennzeichnet, daß die nicht-gewobenen Zellulosefasern einen größeren Anteil des absorbierenden Materials bilden, das flüssigkeitsabsorbierende Material befähigt ist, mehr als 15 Gewichtsteile an Flüssigkeit pro Teil des Materials zu absorbieren, und das thermisch ausgehärtete, wärmeaktivierte Bindemittel einen kleineren Anteil des absorbierenden Materials bildet.

2. Ein absorbierendes Produkt nach Anspruch 1, bei dem die Matrixfasern Zellulosefasern allein sind.

3. Ein absorbierendes Produkt nach Anspruch 1, bei dem die Matrixfasern eine Mischung aus Zellulosefasern und synthetischen Stapelfasern sind.

4. Ein absorbierendes Produkt nach Anspruch 3, bei dem die Zellulosefasern wenigstens 75 Gewichtsprozent der Matrixfasern bilden.

5. Ein absorbierendes Produkt nach Anspruch 3, bei dem die Matrixfasern aus 95 % Weichholz-Kraftpolymerisat und 5 % Polypropylen bestehen.

6. Ein absorbierendes Produkt nach Anspruch 1, bei dem das Bindemittel ein thermisch aushärtendes Bindemittel ist.

7. Ein absorbierendes Produkt nach Anspruch 1, bei dem das Bindemittel ein thermoplastisches Bindemittel ist.

8. Ein absorbierendes Produkt nach Anspruch 1, bei dem das flüssigkeitsabsorbierende Material in einer Menge bis zu 50 Gewichtsprozent der Matrixfasern angewandt ist.

9. Ein Verfahren zur Herstellung eines absorbierenden Materials nach einem der Ansprüche 1 bis 8, welches umfaßt :

(a) Ausbildung einer Matte aus den nicht-gewobenen Matrixfasern in im wesentlichen trockenem Zustand,

(b) Verteilung des flüssigkeitsabsorbierenden Materials in trockener Form unter die nichtgewobenen Matrixfasern in der Matte,

(c) Einschließung des thermisch aushärtenden Bindemittels in die Matte, wobei das Bindemittel im thermisch ausgehärteten Zustand dazu dient, die Matrixfasern und das flüssigkeitsabsorbierende Material zu einer zusammenhängenden Bahn zu verbinden,

(d) Erwärmung der Zusammensetzung des Schrittes (c) auf eine Temperatur, die ausreicht, um das Bindemittel zu aktivieren, und

(e) thermische Aushärtung des Bindemittels, um die Matrixfasern und das flüssigkeitsabsorbierende Material zu einem zusammenhängenden, absorbierenden Produkt zu verbinden, während die Absorptionsfähigkeit des flüssigkeitsabsorbierenden Materials im wesentlichen aufrechterhalten bleibt.

10. Ein Verfahren nach Anspruch 9, bei dem die Matrixfasern aus einer Mischung aus Zellulose- und synthetischen Fasern bestehen.

11. Ein Verfahren nach Anspruch 9, bei dem der Schritt (d) zusammen mit der Anwendung von Druck ausgeführt wird.

12. Ein Verfahren nach Anspruch 9, bei dem das thermoplastische oder thermisch aushärtende Bindemittel mit den Matrixfasern vermischt wird, um aus ihnen eine Matte zu bilden.

13. Ein Verfahren nach Anspruch 13, bei dem das Bindemittel ein fasriges Bindematerial ist.

14. Ein Verfahren nach Anspruch 12, bei dem das Bindemittel ein pulvriges Material ist.

15. Ein Verfahren nach Anspruch 9, bei dem ein thermoplastisches Bindemittel angewandt wird.

16. Ein Verfahren nach Anspruch 9, bei dem ein thermisch aushärtendes Bindemittel angewandt wird.

17. Ein Verfahren nach Anspruch 16, bei dem das Bindemittel in einem flüssigen Trägermittel auf die Matrixfasern und flüssigkeitsabsorbierendes Material enthaltende Matte angewandt wird.

18. Ein Verfahren nach Anspruch 9, bei dem im Schritt (a) eine Matte dadurch gebildet wird, daß die Matrixfasern an einer oder zwei Stellen auf eine bahnbildende Vorrichtung abgelegt werden.

19. Ein Verfahren nach Anspruch 18, bei dem ein flüssigkeitsabsorbierendes Material unter die Matrixfasern in der Matte an einer Stelle zwischen den Stellen verteilt wird, an denen die Matrixfasern abgelegt werden.

## Revendications

1. Matière absorbante formée à sec, sous forme d'une bande cohérente non stratifiée contenant une matrice de fibres cellulosiques non tissées ou d'un mélange de ces fibres avec des fibres synthétiques, une matière absorbant les liquides et un liant activé par la chaleur, thermodurci, la matière absorbant les liquides étant distribuée dans la totalité de ladite matrice et étant liée à cette matrice par le liant activé par la chaleur, à l'état thermodurci caractérisée en ce que les fibres cellulosiques non tissées représentent une proportion dominante de la matière absorbante, la matière absorbant les liquides est capable d'absorber plus de 15 parties en poids de liquide par partie de matière absorbante, et le liant activé par la chaleur, thermodurci, représente une faible proportion de la matière absorbante.

2. Produit absorbant suivant la revendication 1, dans lequel les fibres de la matrice sont constituées uniquement de fibres cellulosiques.

3. Produit absorbant suivant la revendication 1, dans lequel les fibres de la matrice sont constituées d'un mélange de fibres cellulosiques et de fibres synthétiques coupées.

4. Produit absorbant suivant la revendication 3, dans lequel les fibres cellulosiques représentent au moins 75 % en poids des fibres de la matrice.

5. Produit absorbant suivant la revendication 3, dans lequel les fibres de la matrice sont constituées de 95 % de polymère kraft de bois résineux et de 5 % de polypropylène.

6. Produit absorbant suivant la revendication 1, dans lequel le liant est une matière thermodurcissable.

7. Produit absorbant suivant la revendication 1, dans lequel le liant est une matière thermoplastique.

8. Produit absorbant suivant la revendication 1, dans lequel la matière absorbant les liquides est utilisée en une quantité représentant jusqu'à 50 % en poids des fibres de la matrice.

9. Procédé de production d'une matière absorbante suivant l'une des revendications 1 à 8, qui consiste :

(a) à former pratiquement à l'état sec un mat à partir des fibres non tissées de la matrice,

(b) à distribuer à l'état sec parmi les fibres non tissées de la matrice présentes dans ledit mat la matière absorbant les liquides,

(c) à incorporer audit mat le liant thermodurcissable qui, à l'état thermodurci, a pour rôle de lier lesdites fibres de la matrice et la matière absorbant les liquides en une bande cohérente,

(d) à chauffer la composition de l'étape (c) à une température suffisante pour activer ledit liant, et

(e) à provoquer le thermodurcissement dudit liant pour lier lesdites fibres de la matrice et ladite matière absorbant les liquides en un produit absorbant cohérent, tout en conservant effectivement la capacité d'absorption de ladite matière absorbant les liquides.

10. Procédé suivant la revendication 9, dans lequel les fibres de la matrice sont constituées d'un mélange de fibres cellulosiques et de fibres synthétiques.

11. Procédé suivant la revendication 9, dans lequel l'étape (d) est mise en œuvre conjointement avec l'application d'une pression.

12. Procédé suivant la revendication 9, dans lequel le liant thermoplastique ou thermodurcissable est mélangé aux fibres de la matrice pour former un mat.

13. Procédé suivant la revendication 12, dans lequel le liant est une matière fibreuse.

14. Procédé suivant la revendication 12, dans lequel le liant est une matière pulvérisée.

15. Procédé suivant la revendication 9, dans lequel un liant thermoplastique est utilisé.

16. Procédé suivant la revendication 9, dans lequel un liant thermodurcissable est utilisé.

17. Procédé suivant la revendication 16, dans lequel le liant est appliqué au mat contenant les fibres de la matrice et la matière absorbant les liquides dans un véhicule liquide.

18. Procédé suivant la revendication 9, dans lequel, dans l'étape (a), un mat est formé en déposant les fibres de la matrice sur un dispositif de formation de bande à deux ou plus de deux endroits.

19. Procédé suivant la revendication 18, dans lequel une matière absorbant les liquides est répartie parmi les fibres de la matrice présentes dans le mat à un endroit intermédiaire entre les endroits où sont déposées les fibres de la matrice.

# FIG. 1

EP 0 202 472 B1

FIG. 2

UPTAKE EFFICIENCY VS. TIME

100 % EFFICIENCY OF WATER LOCK A-220 POWDER

# FIG. 3

UPTAKE EFFICIENCY VS. TIME

EFFICIENCY OF WATER LOCK A-220 POWDER

GRAMS OF 1% SALINE / GRAM OF POLYMER

MINUTES

FIG. 4

SALINE (1%) ABSORPTION VS. TIME SEC'S

*BASED ON 25.8 GRAMS / m²

TIME SECONDS

(GRAM ABSORBANCE / 12.9 cm²)

(PERCENT OF FREE SWELL)

EP 0 202 472 B1